Europäisches Patentamt

European Patent Office

Office européen des brevets

(11) Publication number: **0 321 764**

A1

(12) **EUROPEAN PATENT APPLICATION**

(21) Application number: 88120264.2

(22) Date of filing: 05.12.88

(51) Int. Cl.4: **A61N 1/37 , A61N 1/365**

(30) Priority: 23.12.87 US 137079

(43) Date of publication of application:
28.06.89 Bulletin 89/26

(84) Designated Contracting States:
DE FR GB IT NL SE

(71) Applicant: Siemens Elema AB
Röntgenvägen 2
S-171 95 Solna 1(SE)

(84) SE

Applicant: Siemens Aktiengesellschaft Berlin
und München
Wittelsbacherplatz 2
D-8000 München 2(DE)

(84) DE FR GB IT NL

(72) Inventor: Andersen, Hans
Strandliden 19
S-16238 Vaellingby(SE)

(54) **Method and means for detecting events in a signal.**

(57) An event detector, useful, for example, in pacemaker technology, detects signal portions which have characteristic events exceeding a certain established threshold. An optimal setting of the detector sensitivity is achieved by passing the input signal through a circuit which measures the signal's maximal values, then establishes the difference between the maximal values and the established threshold, and, in a preferred embodiment, controls the threshold in accordance with the difference and a given desired threshold value. The desired threshold value can be based on, for example, difference histograms or trend data. The event detector can be designed to respond to a variety of signal parameters, such as amplitude and/or slope.

FIG 2

## BACKGROUND OF THE INVENTION

### Field of the Invention

The present invention relates to a method and means for detecting events in an electric signal as well as to a heart pacer comprising such means.

### Description of the Prior Art

Detecting certain characteristic events in electric signals is useful, for example, in the field of pacemaker technology. In this case, a detection element detects depolarization in the atrium or ventricle of a patient's heart. The response of the detection element can then be used as a basis for either triggering or inhibiting pacer stimulation pulses. The triggering threshold chosen for the detection element is especially important, since an inaccurate analysis of the electric signal would lead to a chaotic triggering or unwarranted inhibition of stimulation pulses. The detection element can be designed to respond to a variety of signal parameters, in particular to the amplitude and/or slope of the signal.

Standard pacemaker detection elements are often set by first establishing the level (e.g. amplitude or slope) at which the depolarization signal is barely detected and then simply setting the detection sensitivity to twice this value. This method is, however, relatively inexact; false detection (or lack of detections) may consequently occur. Also, later adjustment of the detection level is usually not undertaken, even though the patient's depolarization signal to the pacemaker can change dramatically.

## SUMMARY OF THE INVENTION

It is an object of the invention to provide a method, means and a heart pacer which allow for a more accurate detection of certain events in a given electric signal. It is a further object of the present invention to provide for an improvement in the detection of such events which are prone to variations in their maximal values. The present invention is defined in the independent claims, and preferable embodiments thereof are defined in the dependent claims.

The above object is achieved in accordance with the principles of the present invention in a method and apparatus for detecting such events, wherein a threshold is established for the signal at a level selected such that an event exceeds the established threshold. The maximum value of the event which exceeds the established threshold is measured, and the difference between the maximum value of the event and the established threshold is calculated. The current established threshold is then reevaluated based at least in part upon the size of the difference, and if necessary, a new threshold is established.

A heart pacemaker embodying the above method and apparatus has means for generating stimulation pulses to the heart of a patient via a pacing electrode connected to the pulse generating means. The pacing electrode may be located in the ventricle or atrium of the heart, or two such electrodes may be used simultaneously in both the ventricle and the atrium. A detection means is provided for detecting events in a depolarization signal originating in the heart indicative of heart activity. The detection means operates as described above, by establishing a threshold for the signal such that an event of interest exceeds the threshold. The maximum value of an event exceeding the established threshold is measured, and the difference between this maximum value and the established threshold is calculated. The size of the difference is used at least in part as a basis for reestablishing a new threshold at a different level, if necessary.

The threshold is also used as a basis for generating a detection signal which is used to control the pulse generator in the heart pacemaker. If the incoming signal exceeds the threshold (which may be established both positively and negatively) this is indicative of natural depolarization of the heart. This causes a detection signal to be generated, which inhibits the pulse generator from generating a stimulation pulse. In the absence of a detection signal, indicating the absence of a natural heart beat, the pulse generator is not inhibited, and generates a stimulation pulse.

The initial detector sensitivity is set in the same manner as is described above for standard pacemakers. An additional circuit is provided, however, which modifies the sensitivity setting (triggering threshold) after evaluating the difference between the maximal value of the electric signal and the previously established sensitivity setting. If the difference is too small, such that a possibility of overlooking characteristic events exists, the sensitivity is increased accordingly. Correspondingly, if the difference is too large, such that noise signals might mistakenly be identified as characteristic events, the sensitivity is decreased. Further evaluation parameters, such as accumulated statistical data (e.g. histograms or trend data), can be additionally supplied to the circuit if desired.

The foregoing and other objects, features and advantages of the invention will be apparent from the following more particular description of a preferred embodiment of the invention, as illustrated in the accompanying drawings.

## BRIEF DESCRIPTION OF THE DRAWINGS

Figure 1 is a schematic block diagram illustrating the principles of the present invention.

Figure 2 is a detailed circuit diagram of a preferred embodiment of an event detector according to the principles of the present invention.

Figure 3 is a pulse diagram for the circuit shown in Figure 2.

Figure 4 is a schematic block diagram of a heart pacer according to the principles of the present invention.

## DESCRIPTION OF THE PREFERRED EMBODIMENTS

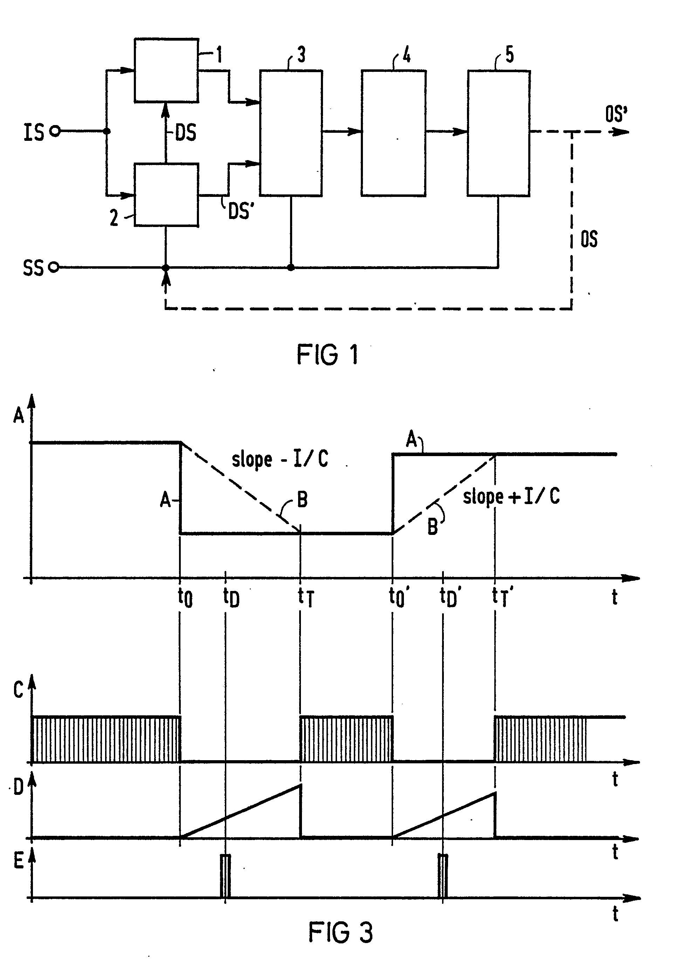

The circuit in Figure 1 shows a maximal value measuring circuit 1, a detector 2, a difference calculation circuit 3, a data establishment circuit 4, and a data evaluation circuit 5, which is preferrably located external to the patient in the case of pacemaker technology. The input signal IS comprises events to be measured by the illustrated circuit, while signal SS defines the sensitivity setting of the circuit and is initially adjusted as described in the introduction.

The input signal IS , for example a depolarization signal received from the heart of a patient, is introduced into the maximal value measuring circuit 1 and the detector 2. Both measuring circuit 1 and detector 2 respond to identical event detection parameters, such as the slope and/or the amplitude of the input signal IS. Whenever a maximal value is detected in input signal IS which is greater than the established threshold of sensitivity setting SS, detector 2 releases a detection signal DS, which activates the maximal value measuring circuit 1. The measuring circuit 1 measures the maximal value of the input signal upon each detection and passes the measured value to the amplitude difference calculation circuit 3. Upon receiving the measured value from the maximal value measuring circuit 1 and a detection signal DS' from the detector 2, the difference calculation circuit 3 calculates the margin between the measured maximal value and the established threshold, given by the sensitivity setting SS. This margin is expressed as a percentage and is given by:

$$M = \frac{P-S}{S} \cdot 100\%$$

where M is the margin,
P is the maximal value measured by the maximal value measuring circuit 1 and
S is the established threshold corresponding to the sensitivity setting.

The data establishment circuit 4 receives the consecutive margin values from the amplitude difference calculation circuit 3 and structures them in various forms, such as histograms, standard deviations, and/or development patterns over time. The data establishment circuit 4 can also be programmed to produce an alarm signal in case the margin shows unfavorable development. The output data of the data establishment circuit 4 is processed by the data evaluation circuit 5, which calculates an optimal sensitivity setting based on the data supplied by the data establishment circuit 4. This sensitivity setting can then be entered as the new triggering threshold, as indicated by broken arrow OS. Alternatively, this sensitivity setting can be simply presented to the doctor (e.g. on a screen), as indicated by broken arrow OS', thereby leaving the new setting to the doctor's discretion.

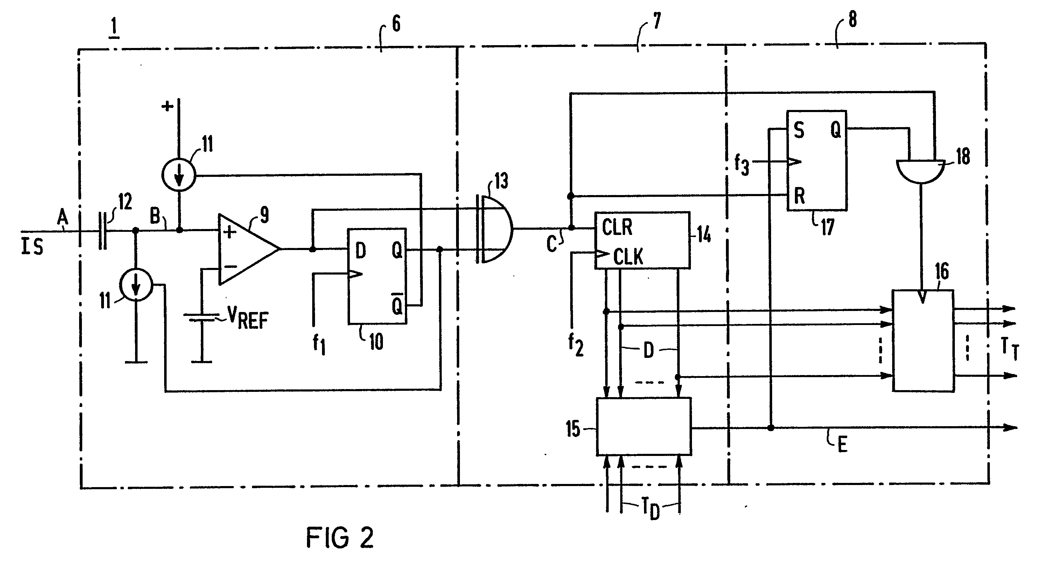

Figure 2 shows a maximal value measuring circuit in a detailed circuit diagram. Digital components are used in this embodiment. The circuit can, however, easily be designed using analog components by one having ordinary skill in the art. The maximal value measuring circuit 1 of Figure 2 is subdivided into a delta modulator 6, an event detector 7 and a storage unit 8. The delta modulator 6 comprises a comparator 9, a D flip-flop 10, current generators 11 and an integration capacitor 12. The inverting input of comparator 9 is connected to a reference voltage $V_{REF}$. The D flip-flop 10 is clocked by a clock frequency $f_1$ (e.g. 16 kHz). The event detector 7 comprises an exclusive-OR gate 13, a counter 14, which is clocked by a frequency $f_2$ (e.g. 8 kHz), and a comparator 15. The comparator 15 compares the output D of the counter 14 with a time reference $T_D$. In case that the output D exceeds the time reference $T_D$, a detector signal E is generated.

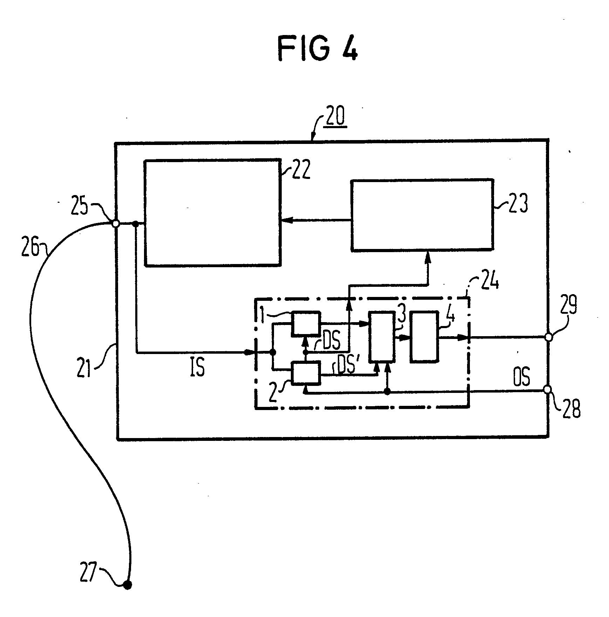

The operation of the circuit of Figure 2 is as follows. The input signal IS, which includes certain characteristic events, is supplied to the delta modulator 6, as indicated in Figure 2. As soon as an event is detected, the opposite side of integration capacitor 12 transfers the signal to the non-inverting input of comparator 9 with a slope of either +I/C or -I/C, whereby I is the current of current generator 11 (programmable) and C is the capacitance of the integration capacitor 12. Whenever the D flip-flop 10 switches from one state to the other, the exclusive-OR gate 13 zeroes the counter 14 in the event detector 7. When the slope of the input signal IS exceeds +I/C or falls below -I/C (namely, upon the occurrence of a high value event), the D flip-flop 10 does not switch. As a result the time counter 14 of event detector 7 is not zeroed. The output D of the time counter 14 increases and meets the time reference $T_D$, causing a detection signal E to be generated, as indicated in Figure 3. The counter 14 continues counting until the D flip-flop 10 is reactivated and a zeroing pulse is transmitted by the exclusive-OR gate 13. However, before zeroing, the counter 14 transfers its peak count value to a register 16 in the storage unit 8, which is clocked via a clocked flip-flop 17 and AND gate 18.

Figure 3 is a pulse diagram depicting signals at various stages in the maximal value measuring circuit 1 of Figure 2. The output signal T of register 16 (see Figure 2) corresponds to the final value attained by counter 14 (D) and is thus given by

$$T_T = t_T - t_c .$$

Since $T_T$ is a measure of the maximal value P, it can be used in conjunction with the time reference $T_D$, which is analogous to the sensitivity setting S and is given by

$$T_D = t_D - t_o,$$

to compute the margin in the difference calculation circuit 3. Thus the detection margin is given by

$$M = \frac{T_T - T_D}{T_D} \cdot 100\%$$

If this margin is too small, i.e., if the output value of register 16 is only slightly larger than the value of the time reference $T_D$, the probability of missing characteristic events is high, indicating that the threshold should be decreased. If the margin is very large, the threshold should be increased, to guard against incorrectly identifying other signal portions as characteristic events.

Figure 4 shows a heart pacer 20 comprising a metallic housing 21, which encapsules electrical components. The illustrated components are a pulse generator 22, a control unit 23, and the detection circuit 24 previously shown in Figure 1. Detection circuit 24 comprises a maximal value measuring circuit 1, a detector 2, a difference calculations circuit 3, and a data establishment circuit 4, as described previously.

The signal output 25 of the pulse generator 22 is electrically connected with the atrium or ventriclel of a patient's heart through pacing lead 26 and pacing electrode 27. The indifferent electrode is formed by the metallic housing 21.

The pacing lead 26 and the pacing electrode 27 together with the housing 21 also serve as detection means for detecting the depolarization signal of the patient's heart. This depolarization signal is input into detection circuit 24 as input signal IS. Signal IS is processed in detection circuit 24, as described in conjunction with Figure 1. Detection signal DS, supplied by detector 2, is additionally transmitted to control unit 23. If control unit 23 receives a detection signal DS from detection circuit 24, it inhibits pulse generator 22 from generating a stimulation pulse. If no detection signal DS is received by control unit 23, no inhibition occurs and a stimulation pulse is generated accordingly by pulse generator 22.

Reference numbers 28 and 29 indicate a signal input and a signal output respectively. Both can be fashioned as, for example, antennas insulated from the housing 21, which receive signals from and transmit signals to components located outside of the patient's body. At 29, a signal is transmitted to an externally located data evaluation circuit 5 (see Figure 1). The data evaluation circuit 5 calculates an optimal sensitivity setting, which is, in turn, transmitted back to the detection circuit 24 and is received by the pacemaker 20 at the signal input 28.

Having thus described the invention with particular reference to the preferred forms thereof, it will be obvious to those skilled in the art to which the invention pertains, after understanding the invention, that various changes and modifications, such as designing the circuit using analog rather than digital components, may be made therein.

## Claims

1. A method for detecting events in an electric signal, comprising the steps of:
   a) establishing a threshold for said signal in a manner that an event exceeds said threshold;
   b') measuring the maximal value of said event exceeding the established threshold;
   c) establishing the difference between the maximal value of said event and the established threshold; and

d) evaluating the threshold dependent on said difference.

2. A method according to claim 1, wherein the difference is expressed as a fraction of said threshold.

3. A method according to claim 1, wherein the threshold is additionally controlled dependent on said difference.

4. A method according to claim 3, wherein the threshold is controlled by said difference and, additionally, by a desired threshold value.

5. A method according to claim 4, wherein said desired value is derived from a histogram of previous difference values.

6. A method according to claim 4, wherein said desired value is derived from a diagram representing the development over time of the previous difference values.

7. Means for detecting events in an electric signal, comprising:

a) means for establishing a threshold for said signal in a manner that an event exceeds that threshold;

b) means for measuring the maximal value of said event exceeding the established threshold,

c) means for establishing the difference between the maximal value of said event and the established threshold; and

d) means for evaluating the threshold dependent on said difference.

8. Means according to claim 7, wherein the difference is expressed as a fraction of said threshold.

9. Means according to claim 7, wherein the evaluating means additionally control the threshold dependent on said difference.

10. Means according to claim 9, wherein the threshold is controlled by said difference, and, additionally, by a desired threshold value.

11. Means according to claim 10, wherein said desired value is derived from a histogram of previous difference values.

12. Means according to claim 10, wherein said desired value is derived from a diagram representing the development over time of the previous difference values.

13. A heart pacer for stimulating the heart of a patient, comprising:

a) generating means for generating stimulation pulses;

b) a pacing electrode means electrically connected with said generating means, said pacing electrode means being designated for being located in the ventricular and/or atrial portion of the heart;

c) detection means for detecting events in a depolarization signal originating in the heart, comprising,

c1) means for establishing a threshold for said signal in a manner that an event exceeds that threshold;

c2) means for measuring the maximal value of said event exceeding the established threshold;

c3) means for establishing the difference between the maximal value of said event and the established threshold: and

c4) means for evaluating the threshold dependent on said difference; and

d) control means for controlling said generating means dependent on a response signal from said detection means.

14. A heart pacer according to claim 13, wherein the evaluating means additionally control the threshold dependent on said difference.

FIG 1

FIG 3

FIG 2

EP 0 321 764 A1

# FIG 4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | US-A-4 708 144 (HAMILTON)<br>* Whole document *<br>--- | 1-14 | A 61 N 1/37<br>A 61 N 1/365 |
| A | FR-A-2 155 989 (SIEMENS)<br>* Whole document *<br>----- | 1,2,7,8 ,13 | |

TECHNICAL FIELDS SEARCHED (Int. Cl.4)

A 61 N

The present search report has been drawn up for all claims

| Place of search | Date of completion of the search | Examiner |
|---|---|---|
| THE HAGUE | 31-03-1989 | LEMERCIER D.L.L. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)